(19) Europäisches Patentamt
European Patent Office
Office européen des brevets

(11) Veröffentlichungsnummer : **0 283 848 B1**

(12) # EUROPÄISCHE PATENTSCHRIFT

(45) Veröffentlichungstag der Patentschrift : **15.05.91 Patentblatt 91/20**

(51) Int. Cl.$^5$ : **C07D 217/04,** C07D 217/20, C07C 209/60

(21) Anmeldenummer : **88103666.9**

(22) Anmeldetag : **09.03.88**

(54) **Verfahren zur Herstellung von Isochinolinen.**

(30) Priorität : **20.03.87 CH 1064/87**

(43) Veröffentlichungstag der Anmeldung : **28.09.88 Patentblatt 88/39**

(45) Bekanntmachung des Hinweises auf die Patenterteilung : **15.05.91 Patentblatt 91/20**

(84) Benannte Vertragsstaaten : **AT BE CH DE FR GB IT LI LU NL SE**

(56) Entgegenhaltungen :
**DE-B- 1 003 211**
**DE-C- 834 103**
**DE-C- 908 138**
**DE-C- 955 769**

(73) Patentinhaber : **F. HOFFMANN-LA ROCHE AG**
**Postfach 3255**
**CH-4002 Basel (CH)**

(72) Erfinder : **Peter, Philippe, Dr.**
**Gebreitenweg 4**
**CH-3930 Visp (CH)**

(74) Vertreter : **Mahé, Jean et al**
**Postfach 3255 Grenzacherstrasse 124**
**CH-4002 Basel (CH)**

**EP 0 283 848 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein Verfahren zur Herstellung der Isochinoline der Formel

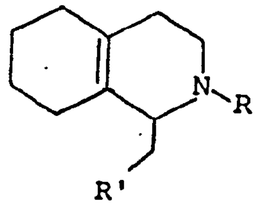

I

worin R Methyl oder Benzyl und R′ Phenyl, p-Hydroxyphenyl oder P-Methoxyphenyl ist, dadurch gekennzeichnet, dass man eine Lösung eines Enamins der Formel

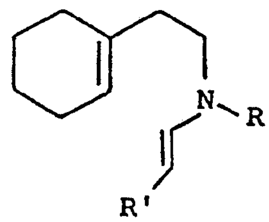

II

worin R und R′ obige Bedeutung haben, in einem Kohlenwasserstoff mit einer wasserfreien Lösung von p-Toluolsulfonsäure in Toluol oder Xylol bei erhöhter Temperatur umsetzt.

Als Kohlenwasserstoff verwendet man zweckmässig aliphatische Kohlenwasserstoffe, wie n-Hexan, oder vorzugsweise aromatische, wie Toluol oder Xylol. Die Reaktion wird zweckmässig zwischen etwa 90 und 115°C, falls R Methyl ist, vorzugsweise bei etwa 110°C und, falls R Benzyl ist, bei etwa 100°C durchgeführt.

Neben dem Isochinolin der Formel I erhält man auch kleine Mengen (4-7%) der isomeren Isochinoline der Formeln

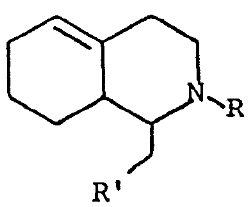

Ia

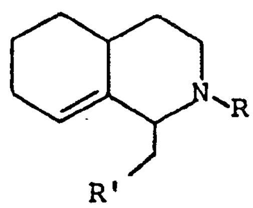

Ib

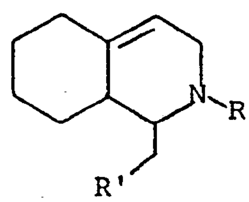

Ic

worin R und R′ obige Bedeutung haben. Erwünschtenfalls kann man diese Nebenprodukte zu den Isochinolinen der Formel I isomerisieren, was unter den gleichen Verfahrensbedingungen wie bei der Ueberführung der Enamine der Formel II in die Isochinoline der Formel I durch Umsetzung mit p-Toluolsulfonsäure bewerkstelligt werden kann.

Die Ausgangsenamine der Formel II können dadurch hergestellt werden, dass man

a) eine Lösung eines Amins der Formel

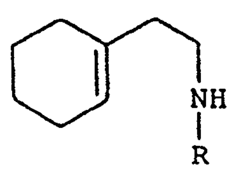

III

worin R Methyl oder Benzyl ist,

in Toluol oder Xylol unter Erwärmen mit einer Lösung eines Aldehyds der Formel $R'CH_2CHO$, worin R' Phenyl, p-Hydrophenyl oder insbesondere p-Methoxyphenyl ist, in Toluol oder Xylol umsetzt, oder

b) eine Lösung des Amins III in einem Kohlenwasserstoff, insbesondere einem aliphatischen Kohlenwasserstoff, wie n-Hexan, unter Erwärmen, gegebenenfalls unter vermindertem Druck, mit einer ätherischen Lösung des Aldehyds $R'CH_2CHO$ umsetzt.

Als Kohlenwasserstoff in der Verfahrensvariante b) kann man einen solchen verwenden, der für die Ueberführung einer Verbindung der Formel II in eine Verbindung der Formel I geeignet ist, vorzugsweise n-Hexan. In der Verfahrensvariante a) wird Wasser und Toluol oder Xylol und in der Verfahrensvariante b) Wasser und Aether abdestilliert.

Die in der Verfahrensvariante a) verwendete Lösung des Aldehyds $R'CH_2CHO$ in Toluol oder Xylol kann man durch Erhitzen auf Rückflusstemperatur einer Suspension eines Alkali- oder Erdalkalimetallsalzes der entsprechenden Glycidsäure der Formel

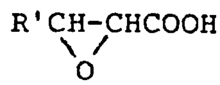

IV

vorzugsweise des Kaliumglycidats, in Toluol oder Xylol in Gegenwart von wässriger Essigsäure erhalten. Die in der Verfahrensvariante b) verwendete ätherische Aldehydlösung kann man durch Behandlung einer Suspension eines Sulfonats der Formel

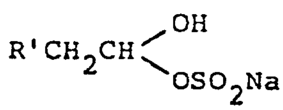

V

in wässrigem Diäthyläther in Gegenwart von Kaliumcarbonat bei einer Temperatur von etwa 1-3°C erhalten.

Die Verbindungen der Formel I kann man, wie z.B. in der schweizerischen Patentschrift Nr. 543514 beschrieben, in Morphinane, wie Dextromethorphan, überführen.

Beispiel 1

a) Zu einem zum Siedepunkt erhitztem Gemisch von 14 g (59 mMol) Kalium-(E)-α,β-epoxy-p-methoxycinnamat, 112 ml Toluol und 28 ml Wasser werden 70 ml einer wässrigen Lösung von 3,64 g Essigsäure getropft. Nach 5 Minuten wird das Gemisch auf Raumtemperatur abgekühlt. Die organische Phase wird mit Wasser und dann mit wässriger Kaliumcarbonatlösung gewaschen. Die Waschwässer werden mit Toluol extrahiert. Die organische Phase wird azeotropisch getrocknet. Die Ausbeute an p-Methoxyphenylacetaldehyd in der erhaltenen Lösung beträgt 92-94%.

b) Die nach Beispiel 1a) hergestellte Lösung von p-Methoxyphenylacetaldehyd in 200 ml Toluol wird unter Rückfluss innerhalb einer Stunde einem Gemisch von 78 g (55,5 mMol) N-Methyl-2-(cyclohexen-1-yl)äthylamin in 20 ml Toluol zugesetzt. Nach 1 Stunde unter Rückfluss erhält man das N-[(E)-p-Methoxystyryl]-N-methyl-2-(cyclohexen-1-yl)äthylamin (Ausbeute 95,3%) gelöst in Toluol.

c) Die nach Beispiel 1b) hergestellte Lösung wird zu einer Lösung von trockener p-Toluolsulfonsäure (entsprechen 60 g Monohydrat) in 600 ml Toluol gegeben. Nach 3-stündigem Erhitzen unter Rückfluss wird das Gemisch abgekühlt und mit 40%iger Natronlauge alkalisch gestellt. Nach Extraktion mit Toluol, Waschen mit Wasser und Einengen der organischen Phase erhält man ein Oel, das bei 190°C unter 1 mbar destilliert wird. Man erhält 13,6 g klares Oel mit einem Gehalt an 1-(p-Methoxybenzyl)-2-methyl-1,2,3,4,5,6,7,8-octahydroisochinolin von 90,8% (Ausbeute 83%) und einem Gehalt an den Isomeren 1-(p-Methoxybenzyl)-2-methyl-1,2,3,4,6,7,8,8a-octahydroisochinolin, 1-(p-Methoxybenzyl)-2-methyl-1,2,3,4,4a,5,6,7-octahydroisochinolin und 1-(p-Methoxybenzyl)-2-methyl-1,2,3,5,6,7,8,8a-octahydroisochinolin von insgesamt 5%.

d) Mittels 4,5 g Oxalsäure in 190 ml Aceton werden 16,3 g Oxalat ausgefällt. Das aus der Mutterlauge isolierte Gemisch von isomeren Isochinolinen wird durch Behandlung mit p-Toluolsulfonsäure wie beschrieben im Beispiel 1c) zum grossen Teil zu 1-(p-Methoxybenzyl)-2-methyl-1,2,3,4,5,6,7,8-octahydroisochinolin isomerisiert. Aus diesem Gemisch werden durch nochmalige Fällung mit 0,56 g Oxalsäure in 15 ml Aceton 0,66 g Oxalat gewonnen. Nach Freisetzung des Oxalats erhält man 12,7 g (Ausbeute 83,5% bezogen auf das Ausgangsamin) mehr als 99% reines1-(p-Methoxybenzyl)-2-methyl-1,2,3,4,5,6,7,8-isochinolin.

Beispiel 2

a) Zu einem auf 1-3°C abgekühlten Gemisch von 8,2 g (33 mMol) Natrium-1-hydroxy-2-(p-methoxyphenyl)äthylsulfonat, 8,4 ml Wasser und 84 ml Diäthyläther werden innerhalb von 5 Minuten 16,8 ml einer wässrigen Lösung von 10 g Kaliumcarbonat zugesetzt. Man lässt 1 Stunde auf Raumtemperatur erwärmen und gibt dann 126 ml Wasser zu. Die organische Phase wird mit Wasser und die wässrige Phase mit Aether gewaschen. Die organische Phase wird dann über Natriumsulfat getrocknet. Die Ausbeute an p-Methoxyphenylacetaldehyd beträgt 57%.

b) Die 125 ml der nach Beispiel 2a) hergestellten ätherischen p-Methoxyphenylacetaldehydlösung werden langsam zu einer Lösung von 3,48 g (16 mMol) N-Benzyl-2-(cyclohexen-1-yl)äthylamin in Hexan bei 50°C gegeben. Das Reaktionswasser und der Aether werden abdestilliert. Nach 4-stündiger Reaktion wird die entstandene Lösung von N-Benzyl-2-(cyclohexen-1-yl)-N-[(E)-p-methoxystyryl]äthylamin bei 100°C zu einer Lösung von 36 g p-Toluolsulfonsäure in 250 ml Toluol gegeben. Nach 2-stündiger Reaktion wird das Gemisch abgekühlt und mit Natronlauge alkalisch gestellt. Die organische Phase wird mit Wasser gewaschen. Die wässrige Phase wird mit Toluol extrahiert. Die organische Phase wird über Natriumsulfat getrocknet, filtriert und eingedampft. Man erhält 5,72 g eines Oels mit einem Gehalt an 2-Benzyl-1-(p-methoxybenzyl)-1,2,3,4,5,6,7,8-octahydroisochinolin von 76,5%. Ausbeute 79% bezogen auf das Ausgangsamin.

## Ansprüche

1. Verfahren zur Herstellung der Isochinoline der Formel

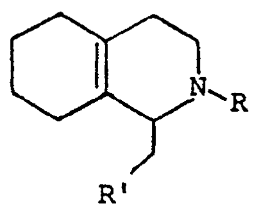

I

worin R Methyl oder Benzyl und R′ Phenyl, p-Hydroxyphenyl oder p-Methoxyphenyl ist,
dadurch gekennzeichnet, dass man eine Lösung eines Enamins der Formel

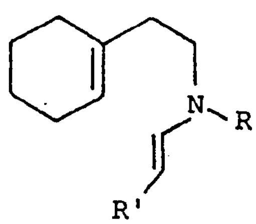

II

worin R und R′ obige Bedeutung haben,
in einem Kohlenwasserstoff mit einer wasserfreien Lösung von p-Toluolsulfonsäure in Toluol oder Xylol bei erhöhter Temperatur umsetzt.

2. Verfahren nach Anspruch 1, dadurch gekennzeichnet, dass R′ p-Methoxyphenyl ist, dass man als Kohlenwasserstoff einen aromatischen Kohlenwasserstoff, wie Toluol oder Xylol, verwendet und dass die Reaktionstemperatur zwischen etwa 90 und 115°C, insbesondere, falls R Methyl ist, etwa 110°C und, falls R Benzyl ist, etwa 100°C beträgt.

3. Verfahren zur Herstellung der Enamine der Formel II nach Anspruch 1, dadurch gekennzeichnet, dass man

a) eine Lösung eines Amins der Formel

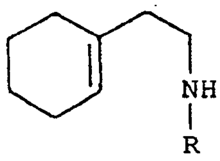

III

worin R Methyl oder Benzyl ist,
in Toluol oder Xylol unter Erwärmen mit einer Lösung eines Aldehyds der Formel $R'CH_2CHO$, worin R' Phenyl, p-Hydrophenyl oder insbesondere p-Methoxyphenyl ist, in Toluol oder Xylol umsetzt, oder
b) eine Lösung des Amins III in einem Kohlenwasserstoff, insbesondere einem aliphatischen, wie n-Hexan, unter Erwärmen, gegebenenfalls unter vermindertem Druck, mit einer ätherischen Lösung des Aldehyds $R'CH_2CHO$ umsetzt.

**Claims**

1. A process for the manufacture of the isoquinolines of the formula

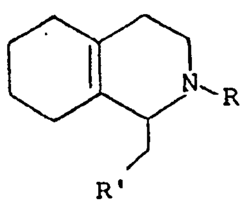

I

wherein R is methyl or benzyl and R' is phenyl,
p-hydroxyphenyl or p-methoxyphenyl,
characterized by reacting a solution of an enamine of the formula

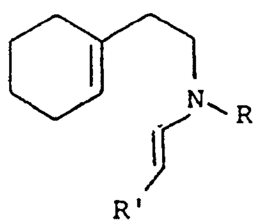

II

wherein R and R' have the above significance,
in a hydrocarbon with an anhydrous solution of p-toluenesulphonic acid in toluene or xylene at an elevated temperature.

2. A process according to claim 1, characterized in that R' is p-methoxyphenyl, in that an aromatic hydrocarbon such as toluene or xylene is used as the hydrocarbon and in that the reaction temperature amounts to between about 90 and 115°C, especially about 110°C where R is methyl and about 100°C where R is benzyl.

3. A process for the preparation of the enamines of formula II according to claim 1, characterized by
a) reacting a solution of an amine of the formula

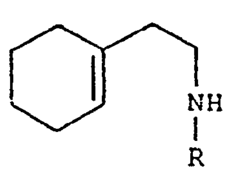

III

wherein R is methyl or benzyl,
in toluene or xylene while heating with a solution of an aldehyde of the formula $R'CH_2CHO$, wherein R' is

phenyl, p-hydrophenyl or especially p-methoxyphenyl, in toluene or xylene, or
b) reacting a solution of the amine III in a hydrocarbon, especially an aliphatic hydrocarbon such as n-hexane, while heating, optionally under reduced pressure, with an ethereal solution of the aldehyde $R'CH_2$ CHO.

## Revendications

1. Procédé de préparation des isoquinoléines de formule

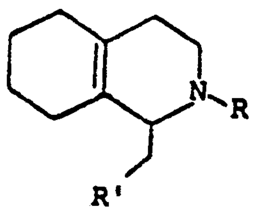

I

dans laquelle R représente un groupe méthyle ou benzyle et R′ un groupe phényle, p-hydroxyphényle ou p-méthoxyphényle,
caractérisé en ce que l'on fait réagir une solution d'une énamine de formule

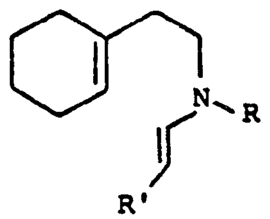

II

dans laquelle R et R′ ont les significations indiquées ci-dessus,
dans un hydrocarbure, avec une solution anhydre d'acide p-toluène-sulfonique dans le toluène ou le xylène à température élevée.

2. Procédé selon la revendication 1, caractérisé en ce que R′ représente un groupe p-méthoxyphényle, l'hydrocarbure utilisé est un hydrocarbure aromatique tel que le toluène ou le xylène, et la température de réaction se situe entre 90 et 115°C environ, ou plus spécialement, lorsque R est un groupe méthyle aux environs de 110°C et lorsque R est un groupe benzyle aux environs de 100°C.

3. Procédé de préparation des énamines de formule II de la revendication 1, caractérisé en ce que
a) on fait réagir une solution d'une amine de formule

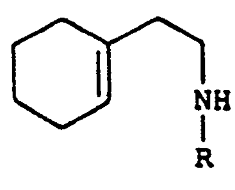

III

dans laquelle R représente un groupe méthyle ou benzyle, dans le toluène ou le xylène, à chaud, avec une solution d'un aldéhyde de formule $R'CH_2$ CHO, dans laquelle R′ représente un groupe phényle, p-hydroxyphényle ou plus spécialement p-méthoxyphényle, dans le toluène ou le xylène, ou bien
b) on fait réagir une solution de l'amine III dans un hydrocarbure, en particulier un hydrocarbure aliphatique tel que le n-hexane, à chaud, éventuellement sous vide, avec une solution éthérée de l'aldéhyde $R'CH_2$ CHO.